(19) Europäisches Patentamt
European Patent Office
Office européen des brevets

(11) **EP 4 414 997 A1**

(12) **EUROPEAN PATENT APPLICATION**

(43) Date of publication:
**14.08.2024 Bulletin 2024/33**

(21) Application number: **23208093.7**

(22) Date of filing: **07.11.2023**

(51) International Patent Classification (IPC):
**G16H 30/40** (2018.01)   **G16H 50/20** (2018.01)

(52) Cooperative Patent Classification (CPC):
**G16H 30/40; G16H 50/20**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **07.02.2023 JP 2023017122**

(71) Applicant: **Canon Medical Systems Corporation Tochigi 324-0036 (JP)**

(72) Inventors:
• **KIMURA, Asateru**
**Otawara-shi, 324-0036 (JP)**
• **SASAKI, Sho**
**Otawara-shi, 324-0036 (JP)**
• **NAKATSUGAWA, Minoru**
**Otawara-shi, 324-0036 (JP)**

(74) Representative: **Marks & Clerk LLP**
**15 Fetter Lane**
**London EC4A 1BW (GB)**

(54) **MEDICAL INFORMATION PROCESSING APPARATUS, MEDICAL INFORMATION PROCESSING METHOD, AND STORAGE MEDIUM**

(57)   According to one embodiment, a medical information processing apparatus includes processing circuitry. The processing circuitry acquires first and second medical images acquired in at least first and second time phases. The processing circuitry inputs the first and second medical images into a gene mutation classification model to generate first and second gene mutation classification results. The processing circuitry judges consistency of the gene mutation classification model based on the first and second gene mutation classification results.

F I G. 1

**Description**

FIELD

Embodiments described herein relate generally to a medical information processing apparatus, a medical information processing method, and a storage medium.

BACKGROUND

Recently, gene mutation classification models which classify the presence or absence of gene mutations from radiation images are known. This type of gene mutation classification model is a non-invasive technique, and therefore, has been attracting attention as a technique for monitoring gene mutations.

On the other hand, such a gene mutation classification model is, according to the inventors' review, not always capable of conducting correct classification, and may conduct oncologically invalid classification during the monitoring. For example, a gene mutation classification model may return a classification that represents a very different tendency or extinction of a gene mutation despite the absence of therapeutic action. Therefore, according to the inventors' study, a method for evaluating the consistency of a gene mutation classification model is needed.

SUMMARY

In relation to the embodiments, the following disclosures are additionally given, which set forth some of the various aspects and optional features of the inventions.

    (1) A medical information processing apparatus including processing circuitry configured to:

        acquire a first medical image and a second medical image acquired in at least a first time phase and a second time phase;
        input the first medical image and the second medical image into a gene mutation classification model to generate a first gene mutation classification result and a second gene mutation classification result; and
        judge consistency of the gene mutation classification model based on the first gene mutation classification result and the second gene mutation classification result.

    (2) The processing circuitry may judge the consistency of the gene mutation classification model based further on medical validity of a gene mutation.
    (3) The processing circuitry may judge the consistency by evaluating a change between the first gene mutation classification result and the second gene mutation classification result based on the validity.
    (4a) The validity may include that: it is medically valid that a tumor acquires a new gene mutation while continuing an existing mutation; it is medically valid that a gene mutation of a tumor continues with increasing complexity; and extinction of a gene mutation in a tumor is rare and not medically valid.
    (4b) The change may represent, for each gene, one of acquisition of a mutation, continuation of no mutation, continuation of a mutation, and extinction of a mutation.
    (5) The processing circuitry may judge the consistency by evaluating a degree of conformance of the change to the validity and calculating a consistency score for each gene.
    (6) If the change is continuation of no mutation or continuation of a mutation, the processing circuitry may calculate the consistency score to have a positive value corresponding to a duration for which no mutation or the mutation has continued.
    (7) If the change is extinction of a mutation, the processing circuitry may calculate the consistency score to have a negative value corresponding to a duration for which the mutation has continued.
    (8) If the change is acquisition of a mutation, the processing circuitry may calculate the consistency score based on a predetermined positive value.
    (9) If the change is acquisition of a mutation and a gene that has acquired the mutation satisfies a predetermined co-occurrence relationship, the processing circuitry may calculate the consistency score to have a value corresponding to a sum of the positive value and a fixed value.
    (10) If the change is acquisition of a mutation and a gene that has acquired the mutation does not satisfy a predetermined exclusivity relationship, the processing circuitry may calculate the consistency score to have a value corresponding to the positive value minus a fixed value.
    (11) If a result of a gene test indicates presence of a mutation while the change is continuation of no mutation or extinction of a mutation, the processing circuitry may calculate the consistency score to have a predetermined maximum negative value.

(12) The processing circuitry may calculate a model consistency score for the gene mutation classification model by summing up the consistency scores for respective genes, and judge the consistency based on the model consistency score and a threshold value.

(13) The processing circuitry may withhold judgment of the consistency if gene mutation treatment is performed between the first time phase and the second time phase.

(14a) The processing circuitry may generate the first gene mutation classification result and the second gene mutation classification result for each of a plurality of gene mutation classification models.

(14b) The processing circuitry may judge the consistency for each of the plurality of gene mutation classification models.

(14c) The processing circuitry may select a gene mutation classification model having said consistency from among the plurality of gene mutation classification models based on a result of judgment of the consistency.

(15) The processing circuitry may generate a medical image by inputting, into an image generation model, a medical image acquired in a time phase corresponding to that of a missing one of the first medical image and the second medical image, and acquire the generated medical image as the missing one of the first medical image and the second medical image.

(16) The processing circuitry may calculate the consistency score by collating a third gene mutation classification result corresponding to a first image feature amount of the first medical image with the first gene mutation classification result and collating a fourth gene mutation classification result corresponding to a second image feature amount of the second medical image with the second gene mutation classification result.

(17) The processing circuitry may calculate the consistency score with a fixed value added if collation results both indicate a match.

(18) The processing circuitry may calculate the consistency score with a fixed value subtracted if at least one of collation results indicates a mismatch.

(19) A medical information processing method including:

acquiring a first medical image and a second medical image acquired in at least the first time phase and a second time phase;

inputting the first medical image and the second medical image into a gene mutation classification model to generate a first gene mutation classification result and a second gene mutation classification result; and

judging consistency of the gene mutation classification model based on the first gene mutation classification result and the second gene mutation classification result.

(20) A medical information processing program for causing a computer to realize:

an acquisition function to acquire a first medical image and a second medical image acquired in at least a first time phase and a second time phase;

a generation function to input the first medical image and the second medical image into a gene mutation classification model to generate a first gene mutation classification result and a second gene mutation classification result; and

a judgment function to judge consistency of the gene mutation classification model based on the first gene mutation classification result and the second gene mutation classification result.

(21) A program for causing a computer to implement each configuration of the medical information processing apparatus.

(22) A non-transitory computer readable storage medium storing the program.

BRIEF DESCRIPTION OF THE DRAWINGS

FIG. 1 is a block diagram showing an example of a medical information processing apparatus according to a first embodiment and its peripheral configuration.

FIG. 2 is a flowchart for explaining operations in the first embodiment.

FIG. 3 is a flowchart for explaining operations of step ST30 in the first embodiment.

FIG. 4 is a schematic diagram for explaining the operations in the first embodiment.

FIG. 5 is a diagram for explaining the operations in the first embodiment.

FIG. 6 is a diagram for explaining the operations in the first embodiment.

FIG. 7 is a diagram for explaining operations in a modification of the first embodiment.

FIG. 8 is a flowchart for explaining operations in a second embodiment.

FIG. 9 is a schematic diagram for explaining the operations in the second embodiment.

FIG. 10 is a block diagram showing an example of a medical information processing apparatus according to a third embodiment and its peripheral configuration.

FIG. 11 is a flowchart for explaining operations in the third embodiment.

FIG. 12 is a schematic diagram for explaining the operations in the third embodiment.

FIG. 13 is a flowchart for explaining operations of step ST10 in a fourth embodiment.

FIG. 14 is a schematic diagram for explaining the operations in the fourth embodiment.

FIG. 15 is a block diagram showing an example of a medical information processing apparatus according to a fifth embodiment and its peripheral configuration.

FIG. 16 is a flowchart for explaining operations in the fifth embodiment.

FIG. 17 is a schematic diagram for explaining operations of step ST33A in the fifth embodiment.

FIG. 18 is a diagram for explaining the operations of step ST33A in the fifth embodiment.

FIG. 19 is a diagram for explaining operations in a modification of the fifth embodiment.

## DETAILED DESCRIPTION

In general, according to one embodiment, a medical information processing apparatus includes processing circuitry. The processing circuitry is configured to acquire a first medical image and a second medical image acquired in at least a first time phase and a second time phase. The processing circuitry is configured to input the first medical image and the second medical image into a gene mutation classification model to generate a first gene mutation classification result and a second gene mutation classification result. The processing circuitry is configured to judge consistency of the gene mutation classification model based on the first gene mutation classification result and the second gene mutation classification result.

Each embodiment will be described using the drawings. In each of the following embodiments, it will be assumed that parts or components with the same reference sign operate in substantially the same way, and duplicate explanations will be omitted as appropriate.

<First Embodiment>

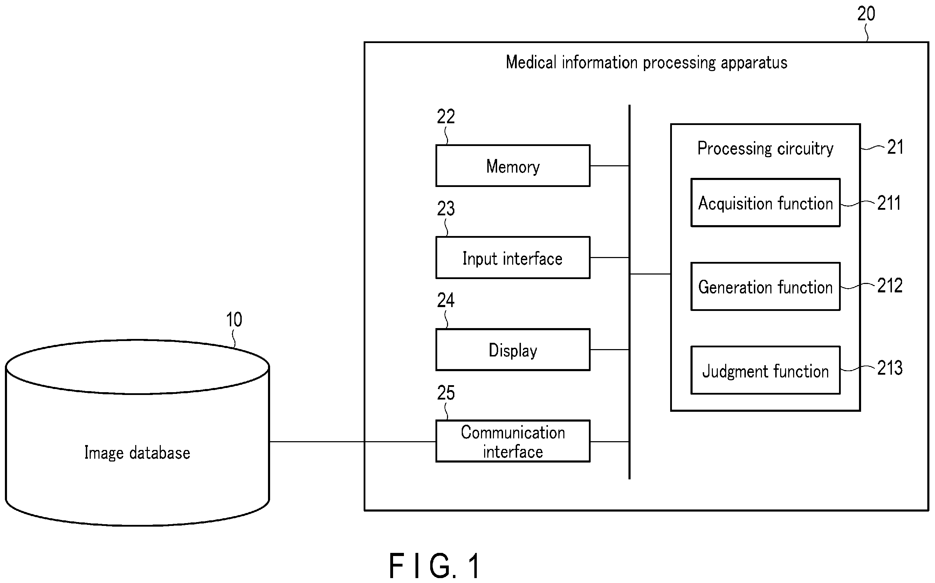

FIG. 1 is a block diagram showing a medical information processing apparatus according to the first embodiment and its peripheral configuration. In FIG. 1, an image database 10 is connected to this medical information processing apparatus 20.

The image database 10 is a storage device that stores digital datasets, such as medical images of a patient and patient information, in association with each other. As the medical images, any modality images including computed tomography (CT) images, magnetic resonance imaging (MRI) images, ultrasound images, and positron emission tomography (PET) images may be used. Each medical image contains, in its accessory information, a time phase indicative of the time and date of acquisition. The patient information is patient-specific information and includes, for example, a patient ID and the name, date of birth, gender, and age of the patient. The image database 10 may further store medical data of the patient. The medical data is information that can be learned by medical practitioners for physical conditions, medical conditions, treatment, etc. of the patient in the course of medical care. The medical data includes, for example, examination history information, image information, electrocardiogram (ECG) information, vital sign information, drug history information, report information, medical record information, and nursing record information. The examination history information is, for example, information indicating the history of test results obtained from specimen tests, bacteriological tests, etc., performed on the patient. The image information is, for example, information indicating the location of a medical image acquired through imaging or the like on the patient. The ECG information is, for example, information on the ECG waveform measured from the patient. The vital sign information is, for example, basic information related to the patient's life. The vital sign information includes, for example, a pulse rate, a respiratory rate, a body temperature, a blood pressure, and a consciousness level. The drug history information is, for example, information indicating the history of drug amounts that have been administered to the patient. The report information is, for example, summarized information on the conditions and disease of the patient which is prepared by an interpreting doctor in a radiology department based on interpretation of medical images such as X-ray images, CT images, MRI images, and ultrasound images, in response to an examination request from a medical doctor in a diagnosis and treatment department. The medical record information is, for example, information input into an electronic medical record by a medical doctor. The medical record information includes, for example, a medical record created at the admission to a hospital, a clinical history of the patient, and a drug prescription history. The nursing record information is, for example, information input into the electronic medical record by a nurse, etc. The nursing record information includes, for example, a nursing record created at the admission to the hospital.

On the other hand, the medical information processing apparatus 20 includes processing circuitry 21, a memory 22, an input interface 23, a display 24, and a communication interface 25.

The processing circuitry 21 reads information, a program, etc., stored in the memory 22 based on an instruction input by a user via the input interface 23, and controls the medical information processing apparatus 20 according to the read information, program, etc. In one example, the processing circuitry 21 is a processor for realizing each of the intended functions according to a program or programs read from the memory 22. Here, examples of the functions include an acquisition function 211, a generation function 212, and a judgment function 213.

The acquisition function 211 acquires a first medical image and a second medical image acquired in at least a first time phase and a second time phase. Note that the acquisition in at least the first time phase and the second time phase means acquisition in multiple time phases. Any medical images may be used as long as they are acquired non-invasively and are classifiable for the presence or absence of a gene mutation for each gene. For example, radiation images, such as CT images, and MRI images are known as such medical images. The acquisition function 211 is an example of an acquisition unit.

The generation function 212 generates a first gene mutation classification result and a second gene mutation classification result by inputting the first medical image and the second medical image into a gene mutation classification model. The generation function 212 is an example of a generation unit.

The judgment function 213 judges the consistency of the gene mutation classification model based on the first gene mutation classification result and the second gene mutation classification result. For example, the judgment function 213 evaluates (judges) the consistency in output of the gene mutation classification model for a focused time phase, using a gene mutation classification result for a medical image of the focused time phase and a gene mutation classification result or results for one or more other time phases, output by the gene mutation classification model. "Consistency" refers to the property of the gene mutation classification model providing outputs (gene mutation classification results) that consistently match medical validity (oncological characteristics, genetic findings, etc.). Consistency is evaluated by comparing multiple gene mutation classification results given by the gene mutation classification model to the known medical validity. In other words, the judgment function 213 may judge the consistency of the gene mutation classification model based further on the medical validity of gene mutations. For example, the judgment function 213 may judge the consistency by evaluating a change between the first and second gene mutation classification results based on the validity. Here, the validity may include, for example, the following (a) through (c).

(a) It is medically valid that a tumor takes on a new gene mutation while continuing an existing gene mutation. This (a) corresponds to, for example, an oncological characteristic wherein a tumor acquires a new gene mutation while inheriting a specific gene mutation.

(b) It is medically valid that a gene mutation of a tumor continues with increasing complexity. This (b) corresponds to, for example, an oncological characteristic wherein a mutation proceeds while increasing its complexity in the form of a driver gene mutation toward a direction favorable to tumor survival.

(c) Extinction of a gene mutation in a tumor is a rare event and is not medically valid. This (c) corresponds to, for example, an oncological characteristic wherein as a tumor develops, driver gene mutations irreversibly increase and so does the overall tumor complexity. It is additionally noted that this (c) corresponds to, for example, circumstances wherein while extinction of a certain driver gene mutation may occur due to competition for survival among tumor cells, it is not regarded as a dominant factor.

It may be assumed that a change between the first and second gene mutation classification results represents, for each gene, one of: acquisition of a mutation, continuation of no mutation, continuation of a mutation, and extinction of a mutation. Here, the "change" may be called a "transition" or a "combination".

The judgment function 213 may judge the consistency by evaluating a degree of conformance of the above change to the validity, and by calculating a consistency score for each gene. For example, if the change is continuation of no mutation or continuation of a mutation, the judgment function 213 may calculate a consistency score having a positive value corresponding to the duration for which no mutation or the mutation has continued. If the change is extinction of a mutation, the judgment function 213 may calculate a consistency score having a negative value corresponding to the duration for which the mutation has continued. If the change is acquisition of a mutation, the judgment function 213 may calculate a consistency score based on a predetermined positive value. The judgment function 213 may calculate a model consistency score, which is for the gene mutation classification model, by summing up the consistency scores for respective genes, so as to judge the consistency based on the model consistency score and a threshold value. The judgment function 213 is an example of a judgment unit.

The memory 22 includes a memory main part for storing electrical information, such as a read only memory (ROM), a random access memory (RAM), a hard disk drive (HDD), and/or an image memory, and peripheral circuitry associated with the memory main part, such as a memory controller and/or a memory interface. The memory 22 stores various programs including a medical information processing program, etc. for the medical information processing apparatus 20, and various data including patient information, medical images, etc. acquired from the image database 10, the gene mutation classification model stored in advance, data being processed, and so on. The medical information processing program may be, for example, a program or programs for causing a computer to realize the acquisition function 211, the generation function 212, and the judgment function 213. The gene mutation classification model is, for example, a trained model that has undergone machine-learning processes with datasets constituted by medical images as input data and gene mutation classification results as output data. This gene mutation classification model outputs gene mutation classification results on a patient based on medical images of the patient. The gene mutation classification results are data that indicates the presence or absence of a gene mutation or mutations for each gene.

The input interface 23 is realized by components for a user to input various instructions, commands, information sets, selections, settings, etc., to the medical information processing apparatus 20, and such components may include a trackball, switch buttons, a mouse, a keyboard, a touchpad which allows input operations through contacting of the operation screen, a touch panel display which integrates a display screen and a touch pad, and so on. The input interface 23 is connected to the processing circuitry 21, etc., and converts input operations received from the user into electrical signals and outputs them to the processing circuitry 21. In the following description of the medical information processing apparatus 20, a "user operation" may be taken to mean an "operation on the input interface 23 by the user". Note that, in the disclosure herein, the input interface 23 is not limited to physical operation components such as a mouse, keyboard, or the like. The examples of the input interface 23 also include processing circuitry for electrical signals that is adapted to receive an electrical signal corresponding to an input operation from an external input device provided separately from the apparatus, and to output this electrical signal to the processing circuitry 21.

The display 24 includes a display main part for displaying given medical images, etc., internal circuitry for supplying display signals to the display main part, and peripheral circuitry including connectors, cables, or the like for connection between the display main part and the internal circuitry. The display 24 is an example of a display unit which displays medical images, etc., under the control of the processing circuitry 21.

The communication interface 25 serves to communicate various data sets between the medical information processing apparatus 20 and the image database 10. Available communication standards include, for example, digital imaging and communications in medicine (DICOM) for communication of medical image information, and Health Level 7 (HL7) for communication of medical text information.

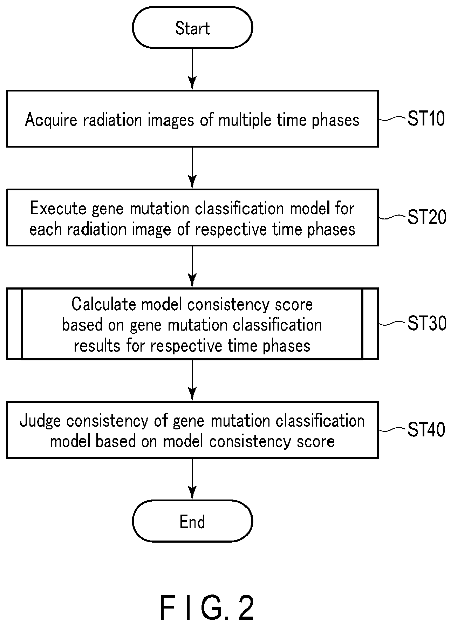

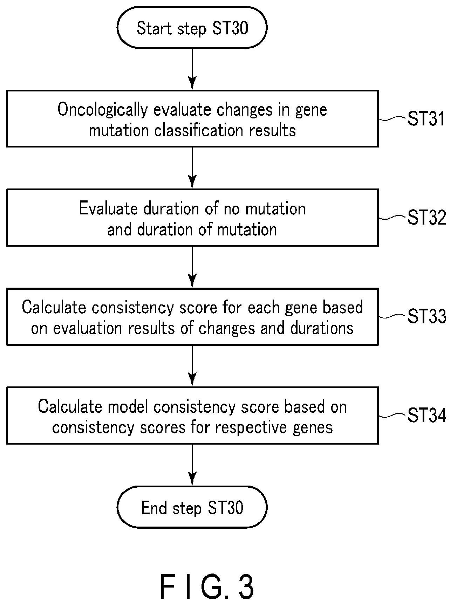

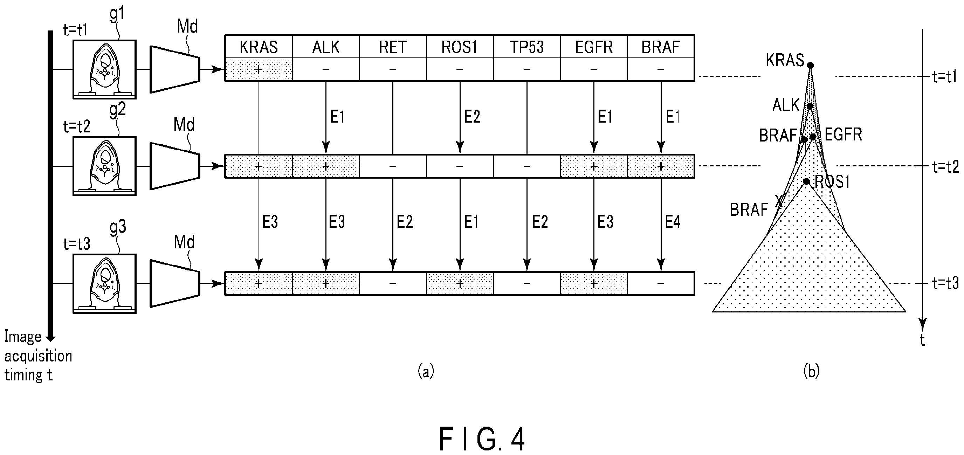

Next, operations of the medical information processing apparatus configured as described above will be explained using the flowcharts in FIGS. 2 and 3 and the schematic diagrams in FIGS. 4 through 6. The following explanation will assume use of radiation images such as CT images as an example of the medical images. However, the medical images are not limited to radiation images; for example, MRI images may also be used.

In step ST10, the processing circuitry 21 of the medical information processing apparatus 20 acquires radiation images of multiple time phases according to a user operation.

After step ST10, in step ST20, the processing circuitry 21 runs the gene mutation classification model in the memory 22 for the radiation image of each time phase. For example, the processing circuitry 21 inputs the radiation image of the first time phase into the gene mutation classification model to generate the first gene mutation classification result. The processing circuitry 21 also generates the second gene mutation classification result by inputting the radiation image of the second time phase into the gene mutation classification model. In the same way, the processing circuitry 21 generates the gene mutation classification results by inputting the radiation images of the respective time phases into the gene mutation classification model.

After step ST20, in steps ST30 to ST40, the processing circuitry 21 judges the consistency of the gene mutation classification model based on each gene mutation classification result. More specifically, the processing circuitry 21 judges the consistency of the gene mutation classification model based further on the medical validity of gene mutations. For example, the processing circuitry 21 calculates the model consistency score which evaluates the validity of gene mutations based on the gene mutation classification result for each time phase (step ST30), and judges the consistency of the gene mutation classification model based on the model consistency score (step ST40). Here, step ST30 is performed through, for example, steps ST31 to ST34 as shown in FIG. 3.

In step ST31, the processing circuitry 21 oncologically evaluates changes in the gene mutation classification results for the respective time phases, for example, in a manner as shown in FIG. 4(a). More specifically, in FIG. 4(a), a gene mutation classification result is generated by the gene mutation classification model Md from each of radiation images g1 to g3 of respective time phases t1 to t3 among image acquisition timings t. Each gene mutation classification result shows an indication of the presence of mutation, "+", or the absence of mutation, "-", for each of seven genes. The signs "+" and "-" may be replaced by other signs such as "1" and "0". Here, changes in the gene mutation classification results between two time phases may be evaluated into four types, namely, E1: acquisition of a mutation, E2: continuation of no mutation, E3: continuation of a mutation, and E4: extinction of a mutation.

For example, the changes in the gene mutation classification results on the genes ALK, EGFR, and BRAF between time phases t1 and t2 are oncologically evaluated as E1: acquisition of a mutation, because these changes represent a transition from the absence of a mutation, "-", to the presence of a mutation, "+". Such an evaluation as E1 is similarly applicable to the case of the gene ROS1 between time phases t2 and t3.

Also, the changes in the gene mutation classification results on the genes RET, ROS1, and TP53 between time phases t1 and t2 are oncologically evaluated as E2: continuation of no mutation, because these changes represent a transition from the absence of a mutation, "-", to the absence of a mutation, "-". Such an evaluation as E2 is similarly applicable to the cases of the genes RET and TP53 between time phases t2 and t3.

The change in the gene mutation classification results on the gene KRAS between time phases t1 and t2 is oncologically evaluated as E3: continuation of a mutation, because this change represents a transition from the presence of a mutation, "+", to the presence of a mutation, "+". Such an evaluation as E3 is similarly applicable to the cases of the genes KRAS, ALK, and EGFR between time phases t2 and t3.

Further, the change in the gene mutation classification results on the gene BRAF between time phases t2 and t3 represents a transition from the presence of a mutation, "+", to the absence of a mutation, "-", and therefore, this change is oncologically evaluated as E4: extinction of a mutation.

The gene mutation classification results for time phases t1 to t3 are schematically expressed as shown in FIG. 4(b). In FIG. 4(b), the vertical axis represents an image acquisition timing t, and the width of the triangle represents a complexity of a mutation according to a survival period. The black dots each represent acquisition of a mutation in a gene, and the cross mark (″X″) represents extinction of a mutation in a gene. Now, as shown in FIG. 4(b), the gene KRAS acquired a mutation before time phase t1 and the mutation has been continuing up to time phase t3. Also, the genes ALK, EGFR, and BRAF acquired mutations between time phases t1 and t2, and the mutations in the genes ALK and EGFR have been continuing up to time phase t3. Also, between time phases t2 and t3, the gene ROS1 acquired a mutation and the mutation in the gene BRAF has disappeared.

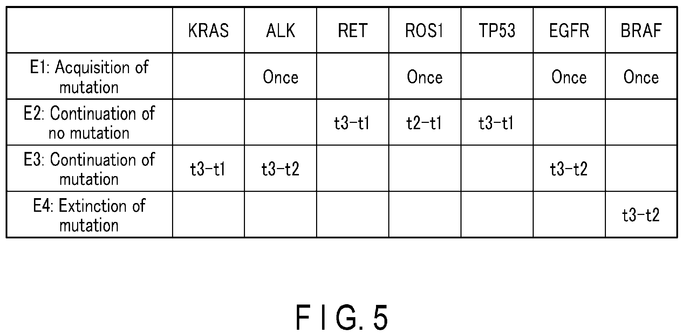

After step ST31, in step ST32, the processing circuitry 21 evaluates the duration of no mutation and the duration of a mutation based on the changes in the gene mutation classification results between two time phases. For example, the processing circuitry 21 calculates the duration of no mutation and the duration of a mutation for each gene as differences between two time phases, as shown in FIG. 5. In the case of extinction of a mutation, the difference (t3-t2) between time phase t2 by which a mutation was acquired and time phase t3 by which the mutation disappeared is calculated as the duration of a mutation in FIG. 5. Also in FIG. 5, since acquisition of a mutation suggests the starting point of a duration of the mutation, the number of times of acquisition is counted.

After step ST32, in step ST33, the processing circuitry 21 calculates a consistency score Gcs_t,n for each gene based on the changes in the gene mutation classification results and the respective durations. Here, the index t represents the image acquisition timing or the time phase of the radiation image. In the examples shown in FIGS. 4 and 5, the consistency score Gcs_t,n is calculated for time phase t3, so t = t3. The index n is the identification number of the gene. In the example shown in FIG. 4, each of the seven genes is identified by a number of n = 1 to n = 7. For example, n = 1 represents the gene KRAS, n = 2 represents the gene ALK, ..., and n = 7 represents the gene BRAF.

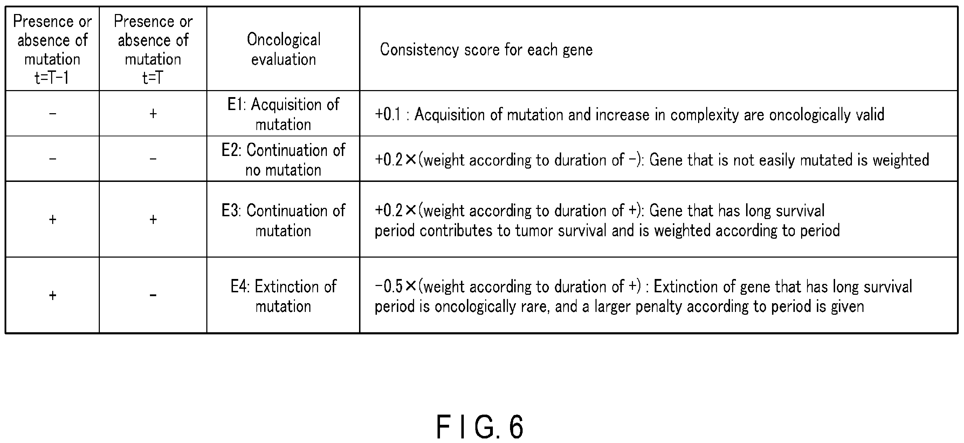

Therefore, the processing circuitry 21 calculates seven consistency scores Gcs_t,1 to Gcs_t,7 according to the oncological evaluations, the durations of no mutation, and the durations of a mutation, as shown in FIGS. 5 and 6. Also, each Gcs_t,n is a value showing evaluation of the degree of conformance of the changes in the genetic mutation classification results to the oncological validity, and it equals, for example, the sum of the scores of E1 to E4. In FIG. 6, "t=T" indicates that the image acquisition timing t is time phase T which is a subject of the consistency judgment. Also, "t=T-1" indicates that the image acquisition timing t is one time phase before the time phase T (i.e., T-1). If the oncological evaluation is E1: the change is acquisition of a mutation, the consistency score is calculated based on a predetermined positive value. If the oncological evaluation is E2: continuation of no mutation or E3: continuation of a mutation, the consistency score is calculated to be a positive value corresponding to the duration of no mutation or the duration of the mutation. If the oncological evaluation is E4: extinction of a mutation, the consistency score is calculated to be a negative value corresponding to the duration of the mutation. However, a score of E1 to E4 that is not applicable is zero. In the case of FIG. 5, the consistency score Gcs_t,1 for the gene KRAS is constituted only by the score of E3 out of E1 to E4. As such, in the case of FIGS. 5 and 6, the consistency score Gcs_t,1 is calculated in the manner as shown in the following equation, where Wa is a weight according to the duration of the mutation.

$$Gcs\_t,1 = 0.2 \times (t3-t1) \times Wa$$

Similarly, the consistency scores Gcs_t,2 to Gcs_t,7 for the respective genes are calculated in the manner as shown in the following equations, where Wb is a weight according to the duration of no mutation.

$$Gcs\_t,2 = 0.1 + 0.2 \times (t3-t2) \times Wa$$

```
Gcs_t,3 = 0.2 × (t3-t1) × Wb
```

```
Gcs_t,4 = 0.1 + 0.2 × (t2-t1) × Wb
```

```
Gcs_t,5 = 0.2 × (t3-t1) × Wb
```

```
Gcs_t,6 = 0.1 + 0.2 × (t3-t2) × Wa
```

```
Gcs_t,7 = 0.1 - 0.5 × (t3-t2) × Wa
```

After step ST33, in step ST34, the processing circuitry 21 calculates the model consistency score based on the consistency scores for the respective genes. For example, the processing circuitry 21 calculates the model consistency score Mcs(t) by summing up the consistency scores Gcs_t,1 to Gcs_t,7 for the respective genes, as shown in the following equation (1).

$$Mcs(t) = \sum_{n=1}^{N} Gcs_{t,n}$$

$$\cdots (1)$$

Upon completion of step ST34, step ST30 is finished.

After step ST30, in step ST40, the processing circuitry 21 judges the consistency of the gene mutation classification model based on the model consistency score and a threshold value. For example, if the model consistency score exceeds the threshold value, the processing circuitry 21 determines that the gene mutation classification model is consistent. After step ST40, the process is terminated.

According to the first embodiment as described above, the processing circuitry 21 acquires a first medical image and a second medical image acquired in at least a first time phase and a second time phase. The processing circuitry 21 inputs the first medical image and the second medical image into the gene mutation classification model Md to generate a first gene mutation classification result and a second gene mutation classification result. The processing circuitry 21 judges consistency of the gene mutation classification model based on the first gene mutation classification result and the second gene mutation classification result. Thus, the consistency of the gene mutation classification model Md can be evaluated based on the gene mutation classification results for multiple time phases. In addition, physicians can evaluate the consistency of the gene mutation classification model, which would facilitate the clinical application of gene mutation classification models. To be more specific, physicians nowadays are not encouraged to clinically apply gene mutation classification models since there is a risk of causing medical accidents due to errors in the estimation of a mutation which may result from an oncologically invalid classification performed by a gene mutation classification model. In contrast, the present embodiment, through the consistency evaluation of the gene mutation classification model Md, allows physicians to consider the possibility of clinical application of the genetic mutation classification model, and therefore, the present embodiment offers a good prospect of the clinical application of gene mutation classification models.

Also according to the first embodiment, the processing circuitry 21 judges the consistency of the gene mutation classification model Md based further on the medical validity of gene mutations. Therefore, in addition to the aforementioned effects, a judgment result supported by the medical validity of gene mutations can be obtained.

According to the first embodiment, the processing circuitry 21 judges the consistency by evaluating a change between the first gene mutation classification result and the second gene mutation classification result based on the validity. Therefore, in addition to the aforementioned effects, a judgment result reflecting the evaluation based on the medical validity can be obtained.

According to the first embodiment, the validity takes into consideration that: it is medically valid that a tumor acquires a new gene mutation while continuing an existing gene mutation; it is medically valid that a gene mutation of a tumor continues with increasing complexity; and extinction of a gene mutation in a tumor is rare and not medically valid. In addition, a change in the gene mutation classification results represents one of the following for each gene:

acquisition of a mutation, continuation of no mutation, continuation of a mutation, and extinction of a mutation. Thus, in addition to the aforementioned effects, such indications of the validity and the contents of the change can further enhance the accuracy of the consistency judgment.

According to the first embodiment, the processing circuitry 21 judges the consistency by evaluating a degree of conformance of the change to the validity and calculating a consistency score Gcs_t,n for each gene. Thus, in addition to the aforementioned effects, the consistency can be judged based on quantitative scores.

According to the first embodiment, if the change is continuation of no mutation or continuation of a mutation, the processing circuitry 21 calculates a consistency score Gcs_t,n having a positive value corresponding to the duration for which no mutation or the mutation has continued. Thus, in addition to the aforementioned effects, it is possible to calculate a consistency score Gcs_t,n having a higher value according to the medically valid durations.

According to the first embodiment, if the change is extinction of a mutation, the processing circuitry 21 calculates the consistency score having a negative value corresponding to the duration for which the mutation has continued. Thus, in addition to the aforementioned effects, it is possible to calculate, in response to the medically invalid extinction of the mutation, a consistency score Gcs_t,n having a lower value according to the duration of the mutation before its extinction.

According to the first embodiment, if the change is acquisition of a mutation, the processing circuitry 21 calculates a consistency score Gcs_t,n based on a predetermined positive value. Thus, in addition to the aforementioned effects, it is possible to calculate a consistency score Gcs_t,n in which the medically valid acquisition of the mutation is quantified.

According to the first embodiment, the processing circuitry 21 calculates a model consistency score Mcs(t) for the gene mutation classification model Md by summing up the consistency scores Gcs_t,n for respective genes, and judges the consistency based on the model consistency score Mcs(t) and a threshold value. Therefore, in addition to the aforementioned effects, the consistency of the gene mutation classification model Md can be judged quantitatively by summing up the consistency scores Gcs_t,n for the respective genes.

(Modifications)

The first embodiment has assumed that the consistency score Gcs_t,n for each gene is calculated from changes in the gene mutation classification results without considering known genetic findings, but the embodiment is not limited to this. For example, the processing circuitry 21 may consider genetic findings such as results of gene tests, co-occurrence/exclusivity relationships of gene mutations, etc. A gene test is a test which can determine mutations in about one to five genes. That is, a gene or genes for the gene test would serve the purpose if the gene or the genes overlap at least one of the multiple genes for which the gene mutation classification results are obtained.

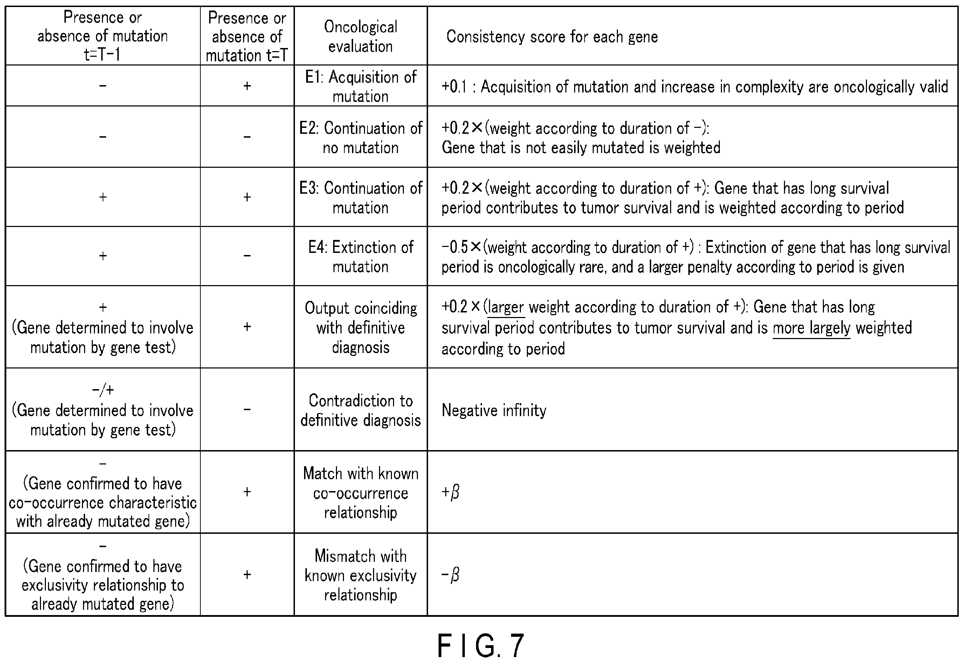

For example, the processing circuitry 21 may calculate the consistency score Gcs_t,n for each gene to be a predetermined maximum negative value if the result of a gene test indicates the presence of a mutation while the above-discussed change is continuation of no mutation or extinction of a mutation. In other words, as shown in FIG. 7, the third row from the bottom, the processing circuitry 21 calculates the consistency score for each gene so that it has the maximum negative value if the change in the gene mutation classification results contradicts the result of the gene test which is a definitive diagnosis. According to such a modification, a further effect can be attained in addition to the aforementioned effects. Namely, the consistency score for each gene is set to the maximum negative value if the change contradicts the result of the gene test, which renders the model consistency score Mcs(t) equal to or below the threshold value even after summation with the consistency scores for the other genes. Therefore, the gene mutation classification model can be determined to be lacking consistency in the event of a contradiction to the result of the gene test.

Also for example, as shown in FIG. 7, the fourth row from the bottom, the processing circuitry 21 may calculate the consistency score Gcs_t,n for each gene so that it has a positive value with a larger weight according to the duration of a mutation if the change in gene mutation classification results matches the result of a gene test. According to such a modification, a further effect can be attained in addition to the aforementioned effects. Namely, the consistency score for each gene is set to be a larger positive value if the change matches the result of the gene test, which increases the value of the model consistency score Mcs(t). Therefore, the probability that the gene mutation classification model will be determined to be consistent can be increased in the case of the result of the gene test being matched.

For example, as shown in FIG. 7, the second row from the bottom, the processing circuitry 21 may calculate the consistency score Gcs_t,n for each gene so that it has a value corresponding to the sum of the positive value and a given fixed value $\beta$ if the above-discussed change is acquisition of a mutation and the gene that has acquired the mutation satisfies a predetermined co-occurrence relationship. More specifically, supposing an instance where a gene G1 already caused a mutation and a gene G2 which has been confirmed to have a co-occurrence characteristic with the gene G1 acquires a mutation, it is regarded that the gene G2 that has acquired the mutation satisfies the predetermined co-occurrence relationship. Therefore, the consistency score for each gene is increased by a given value in the case of the predetermined co-occurrence relationship being met, and the probability that the gene mutation classification model will be determined to be consistent can be increased.

For example, as shown in FIG. 7, the first row from the bottom, the processing circuitry 21 may calculate the consistency score Gcs_t,n for each gene so that it has a value corresponding to the positive value minus the fixed value β if the above-discussed change is acquisition of a mutation and the gene that has acquired the mutation does not satisfy a predetermined exclusivity relationship. More specifically, supposing an instance where a gene G1 already caused a mutation and a gene G2 which has been confirmed to have an exclusivity relationship to the gene G1 acquires a mutation, it is regarded that the gene G2 that has acquired the mutation does not satisfy the predetermined exclusivity relationship. Therefore, the consistency score for each gene is decreased by a given value in the case of the predetermined exclusivity relationship not being met, and the probability that the gene mutation classification model will be determined to be consistent can be decreased.

<Second Embodiment>

In addition to the configurations of the first embodiment, the second embodiment adopts a configuration to take into account the fact of treatment of a genetic mutation for determining the consistency of the gene mutation classification model.

Accordingly, the judgment function 213 of the processing circuitry 21, in addition to the functional features described above, withholds the judgment of the consistency if treatment of a gene mutation has been performed between the first and second time phases. More specifically, since treatment of a gene mutation extinguishes the gene mutation, the gene mutation classification result after the treatment of the gene mutation is deviated. This is true even for the gene mutation classification results given by a gene mutation classification model that has consistency. For this reason, the judgment of the consistency of the gene mutation classification model is withheld after the treatment of the gene mutation.

Other configurations are the same as those in the first embodiment.

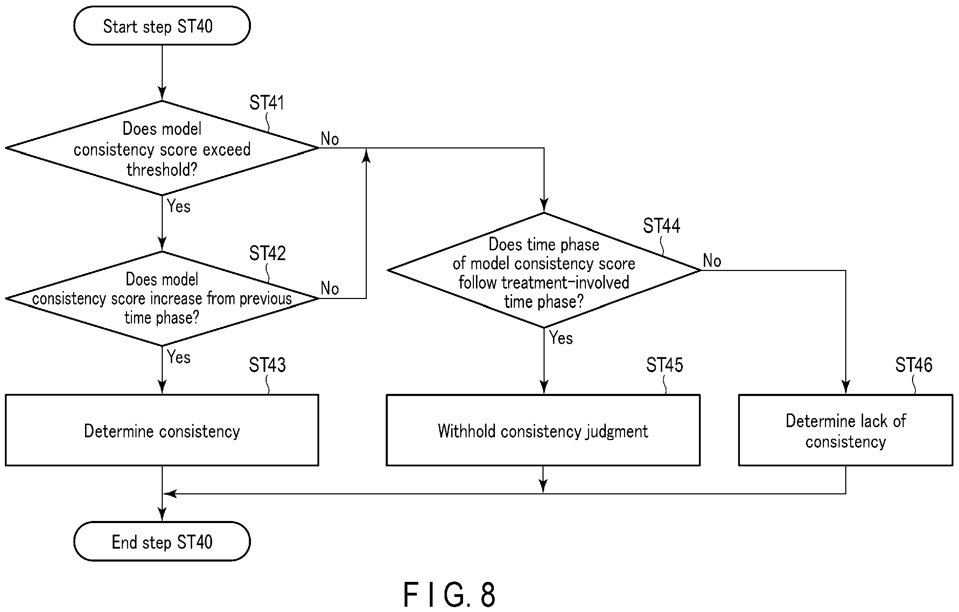

According to the above configuration, after the model consistency score Mcs(t) is calculated by executing steps ST10 to ST34 as described above, step ST40 as shown in FIG. 8 is started. Step ST40 includes steps ST41 to ST46.

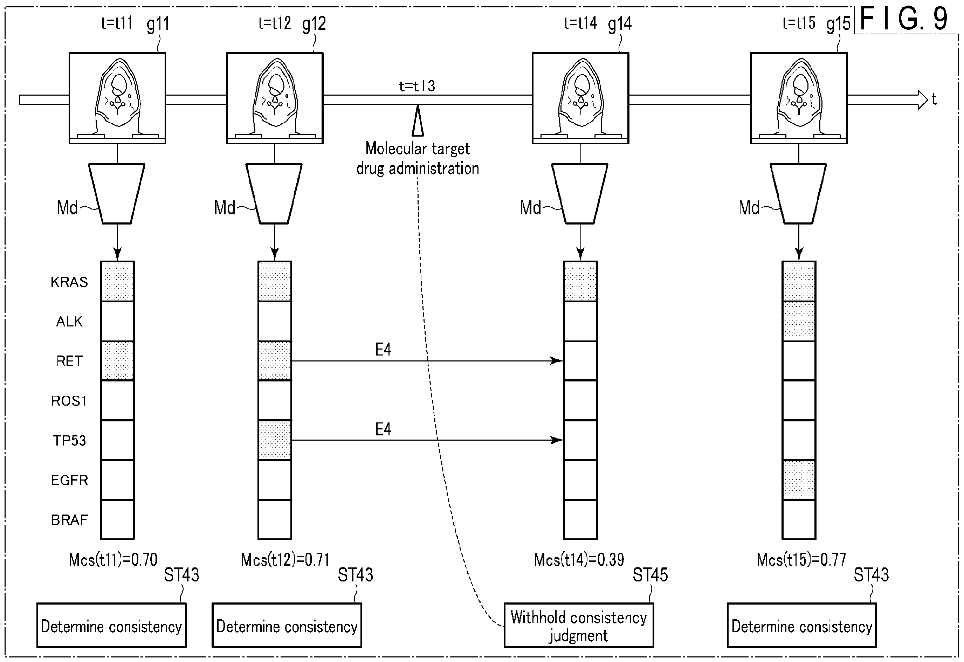

In step ST41, the processing circuitry 21 determines whether or not the model consistency score Mcs(t) exceeds the threshold value, and if it does, the processing circuitry 21 moves to step ST42. For example, as shown in FIG. 9, the processing circuitry 21 generates respective gene mutation classification results from radiation images g11, g12, g14, and g15 of respective time phases t11, t12, t14, and t15 by the gene mutation classification model Md. The model consistency scores Mcs(t11), Mcs(t12), Mcs(t14), and Mcs(t15) for the respective time phases are also generated from the changes in each gene mutation classification result. Assuming that a threshold α of 0.6 is used, the processing circuitry 21 moves to step ST42 upon making a determination for any of the model consistency scores Mcs(t11), Mcs(t12), and Mcs(t15). If the result of the determination in step ST41 is negative, then step ST44 is the next step. For example, upon making a determination for the model consistency score Mcs(t14), the processing circuitry 21 moves to step ST44.

After step ST41, in step ST42, the processing circuitry 21 determines whether or not the model consistency score Mcs(T) for time phase T has increased from the model consistency score Mcs(T-1) for the previous time phase (T-1), and if it has increased, the processing circuitry 21 moves to step ST43. For example, upon making a determination for any of the model consistency scores Mcs(t11), Mcs(t12), and Mcs(t15), the processing circuitry 21 moves to step ST43. If the result of the determination in step ST42 is negative, then step ST44 is the next step.

After step ST42, in step ST43, the processing circuitry 21 determines that the gene mutation classification model corresponding to the model consistency score Mcs(T) for time phase T is consistent. For example, the processing circuitry 21 determines that the gene mutation classification model Md corresponding to any of the model consistency scores Mcs(t11), Mcs(t12), and Mcs(t15) is consistent. After step ST43, the process is terminated.

On the other hand, in step ST44, the processing circuitry 21 determines whether or not the model consistency score Mcs(T) is a model consistency score for time phase T that follows the time phase (T-1) in which treatment has been performed, and if the result of the determination is positive, the processing circuitry 21 moves to step ST45. For example, the model consistency score Mcs(t14) is a model consistency score for time phase t14 that follows time phase t13 in which treatment with the administration of a molecular target drug has been performed, and therefore, the processing circuitry 21 in this case moves to step ST45. If the result of the determination in step ST44 is negative, the processing circuitry 21 moves to step ST46.

In step ST45, the processing circuitry 21 withholds the judgment of the consistency of the gene mutation classification model Md corresponding to the model consistency score Mcs(T) for time phase T that follows the time phase (T-1) involving the treatment. For example, the changes from the gene mutation classification result for time phase t12 to the gene mutation classification result for time phase t14 show extinction of a mutation (E4) in the genes RET and TP53, which at first glance would give a determination of lack of consistency. However, the extinction of a mutation (E4) seen between time phases t12 and t14 may have been attributable to the treatment, i.e., the administration of a molecular target drug, in the intermediate time phase t13. Therefore, the processing circuitry 21 withholds the consistency judgment that uses the model consistency score Mcs(t14) for time phase t14 following time phase t13, in which the treatment with the administration of a molecular target drug has been performed. After step ST45, the process is terminated.

In step ST46, the processing circuitry 21 determines that the gene mutation classification model corresponding to the model consistency score Mcs(T) for time phase T is not consistent. After step ST46, the process is terminated.

According to the second embodiment as described above, the processing circuitry 21 withholds the consistency judgment if treatment of a gene mutation has been performed between the first time phase and the second time phase. Therefore, in addition to the aforementioned effects, it is possible to exclude the influence of the treatment effect in the instances where treatment of gene mutations has been performed, allowing for more accurate consistency evaluation of the gene mutation classification model.

<Third Embodiment>

In addition to the configurations of the first or the second embodiment, the third embodiment adopts a configuration of selecting an appropriate gene mutation classification model from among multiple gene mutation classification models.

Accordingly, the generation function 212 of the processing circuitry 21, in addition to the functional features described above, generates a first gene mutation classification result and a second gene mutation classification result for each of the multiple gene mutation classification models.

The judgment function 213 of the processing circuitry 21, in addition to the functional features described above, judges the consistency of each of the multiple gene mutation classification models.

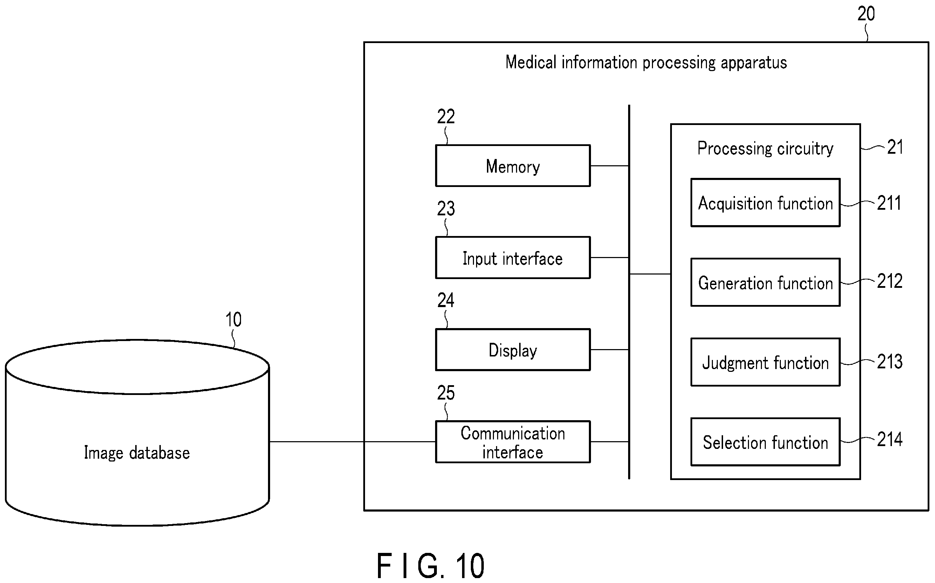

Also, in addition to the configuration described above, the processing circuitry 21 has a selection function 214, as shown in FIG. 10. The selection function 214 selects a gene mutation classification model having said consistency from among the multiple gene mutation classification models, based on the results of the consistency judgment. The selection function 214 is an example of a selection unit.

Other configurations are the same as those in the first or the second embodiment.

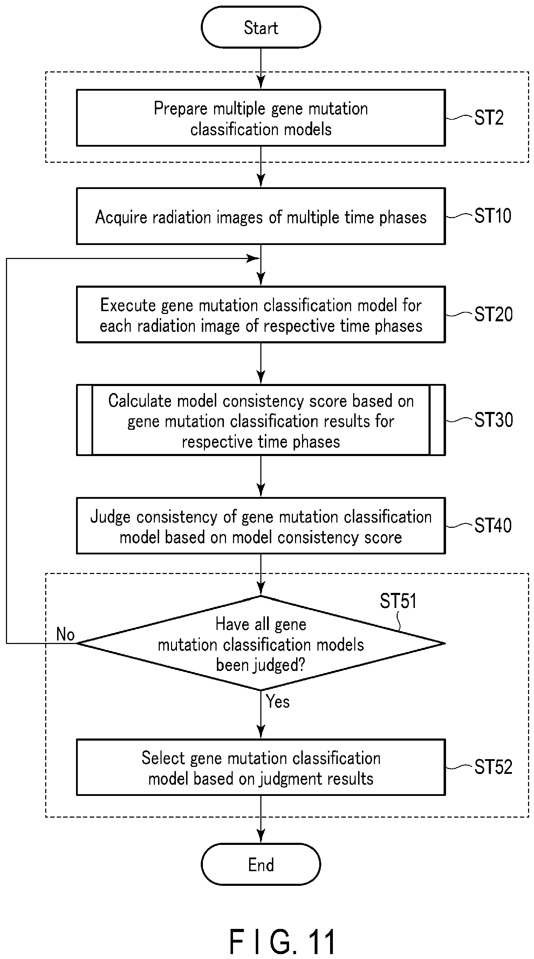

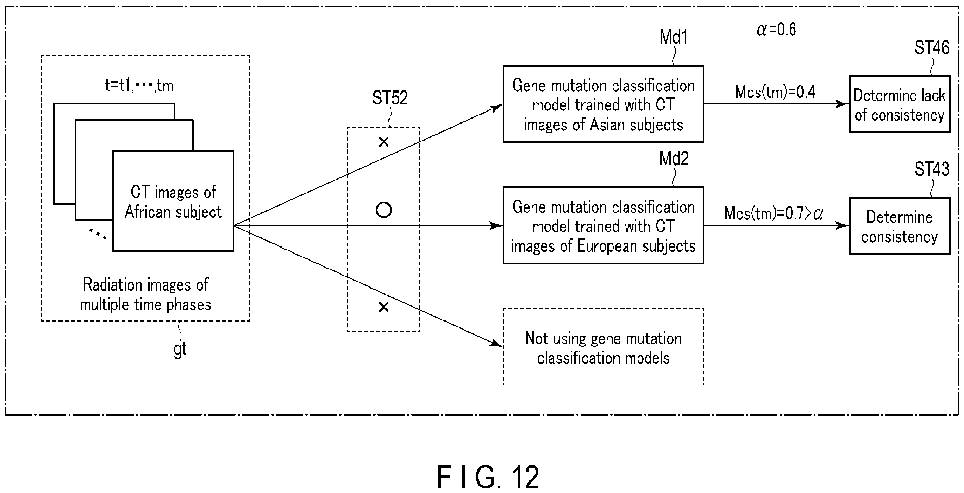

Next, operations of the medical information processing apparatus configured as described above will be explained using the flowchart in FIG. 11 and the schematic diagram in FIG. 12.

In step ST2, the processing circuitry 21 prepares multiple gene mutation classification models. In one example, the processing circuitry 21 acquires multiple gene mutation classification models Md1 and Md2 as shown in FIG. 12 from an external server or the like and stores them in the memory 22. For example, the gene mutation classification model Md1 is a trained model that has been trained with CT images of Asian patients. The gene mutation classification model Md2 is a trained model that has been trained with CT images of European patients.

After step ST2, in step ST10, the processing circuitry 21 acquires radiation images gt of multiple time phases t1 to tm according to a user operation or operations. For example, the radiation images gt are CT images of an African patient.

After step ST10, in step ST20, the processing circuitry 21 runs one of the gene mutation classification models in the memory 22 for the radiation images gt of the respective time phases t1 to tm.

After step ST20, in step ST30, the processing circuitry 21 calculates a model consistency score which evaluates the validity of genetic mutations based on the gene mutation classification result for each time phase.

After step ST30, in step ST40, the processing circuitry 21 judges the consistency of the gene mutation classification model based on the model consistency score.

After step ST40, in step ST51, the processing circuitry 21 determines whether or not all the prepared gene mutation classification models Md1 and Md2 have been subjected to the judgment. If the result of this determination is no, the processing circuitry 21 returns to step ST20 and repeats the processing steps ST20 to ST51 for one of the gene mutation classification models that have not been subjected to the judgment. On the other hand, if, as a result of the determination in step ST51, it is found that all the gene mutation classification models Md1 and Md2 have been subjected to the judgment, the processing circuitry 21 moves to step ST52.

After step ST51, in step ST52, the processing circuitry 21 selects a gene mutation classification model based on the judgment result for each of the gene mutation classification models. For example, as shown in FIG. 12, it will be supposed that the result of judgment of the gene mutation classification model Md1 indicates lack of consistency, and the result of judgment of the gene mutation classification model Md2 indicates consistency. In this case, the processing circuitry 21 selects the gene mutation classification model Md2 showing consistency. Note that if all the gene mutation classification models Md1 and Md2 lack consistency, the processing circuitry 21 selects not using the gene mutation classification models. If each of the gene mutation classification models Md1 and Md2 has consistency, the processing circuitry 21, for example, selects one of the gene mutation classification models that has a higher model consistency score Mcs(tm). In any case, the process is terminated after step ST52.

According to the third embodiment as described above, the processing circuitry 21 generates a first gene mutation classification result and a second gene mutation classification result for each of multiple gene mutation classification models. The processing circuitry 21 judges the consistency of each of the multiple gene mutation classification models. The processing circuitry 21 selects a gene mutation classification model having said consistency from among the multiple gene mutation classification models, based on the results of the consistency judgment. Therefore, in addition to the aforementioned effects, an appropriate gene mutation classification model can be selected from among the multiple gene mutation classification models. For example, if there are multiple gene mutation classification models for the same gene, and which model is appropriate to deal with an input is not apparent, performing the consistency evaluation of each gene mutation classification model enables selection of an appropriate gene mutation classification model.

<Fourth Embodiment>

In addition to the configurations of any of the first, the second, and the third embodiments, the fourth embodiment adopts a configuration to, if a set of medical images of multiple time phases is not available, generate a missing medical image of a time phase from another modality image.

Accordingly, the acquisition function 211 of the processing circuitry 21, in addition to the functional features described above, generates a medical image by inputting an ultrasound image acquired in the time phase corresponding to the time phase of the missing one of the first and the second medical images, into an image generation model. The acquisition function 211 here acquires the generated medical image as the missing medical image.

Other configurations are the same as those of any of the first to third embodiments.

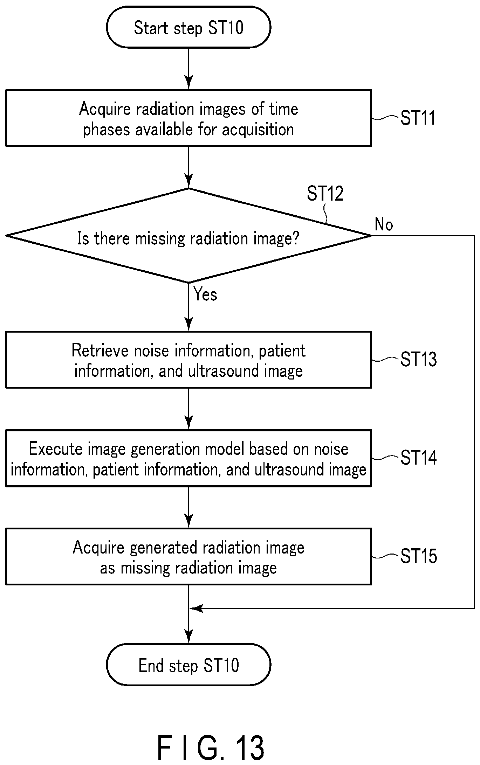

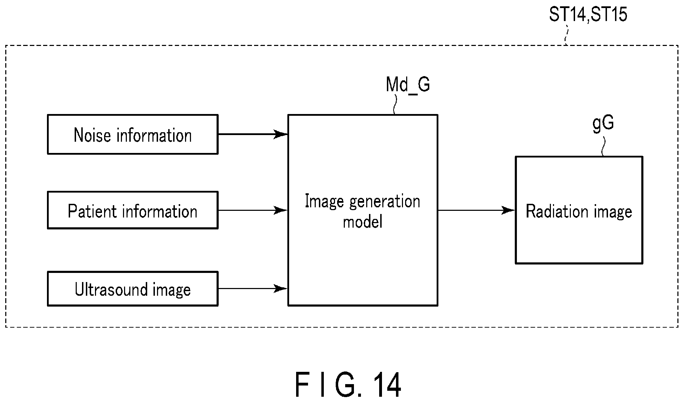

Next, operations of the medical information processing apparatus configured as described above will be explained using the flowchart in FIG. 13 and the schematic diagram in FIG. 14.

The processing circuitry 21 performs step ST10 to acquire radiation images of multiple time phases. Step ST10 includes steps ST11 to ST15.

In step ST11, the processing circuitry 21 acquires radiation images of the time phases available for acquisition.

After step ST11, in step ST12, the processing circuitry 21 determines whether or not there are missing radiation images, and if not, the processing circuitry 21 skips steps ST13 to ST15 and finishes step ST10. On the other hand, if the result of the determination in step ST12 shows a missing radiation image or images, the processing circuitry 21 moves to step ST13.

After step ST12, in step ST13, the processing circuitry 21 retrieves noise information, patient information, and an ultrasound image from the memory 22 for the time phase of each missing radiation image. The noise information is information indicating noise in the ultrasound image. The patient information is information indicating the patient who was the subject of the acquired radiation images. The ultrasound image here is a medical image of the patient acquired by an ultrasound diagnostic apparatus (i.e., another modality).

After step ST13, in step ST14, the processing circuitry 21 runs the image generation model Md_G in the memory 22 based on the noise information, the patient information, and the ultrasound image as shown in FIG. 14. For example, the processing circuitry 21 generates a radiation image gG by inputting the noise information, the patient information, and the ultrasound image into the image generation model Md_G.

After step ST14, in step ST15, the processing circuitry 21 acquires the generated radiation image gG as the missing radiation image. This completes step ST10, constituted by steps ST11 to ST15.

After completion of step ST10, the processing circuitry 21 performs step ST20 and the subsequent steps in the same manner as described above.

According to the fourth embodiment as described above, the processing circuitry 21 generates a medical image by inputting a medical image acquired in the time phase corresponding to the time phase of the missing one of the first and the second medical images, into the image generation model, and the processing circuitry 21 acquires this generated medical image as the missing medical image. Therefore, in addition to the aforementioned effects, missing medical images can be generated from other modality images even if a set of medical images of multiple time phases is not available.

<Fifth Embodiment>

In addition to the configurations of any of the first to fourth embodiments, the fifth embodiment adopts a configuration to more accurately calculate the consistency score for each gene using one or more image feature amounts which can be calculated mechanically from medical images.

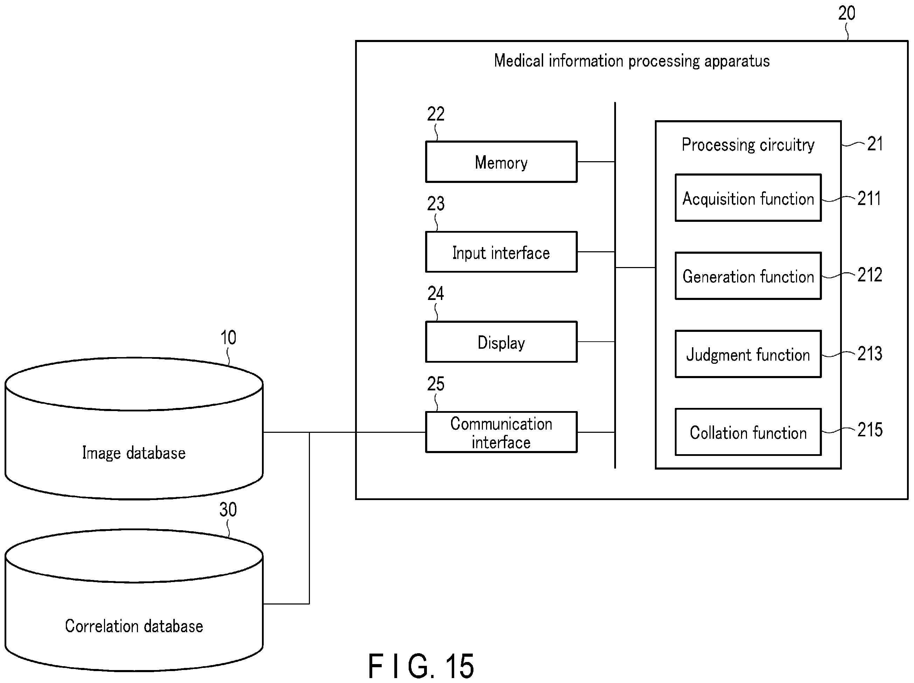

Accordingly, the medical information processing apparatus 20 is, for example, further connected to a correlation database 30 as shown in FIG. 15. The correlation database 30 is a storage device storing an image feature amount of a medical image in association with a gene mutation that has a correlation with the image feature amount. Not limited to this, the correlation database 30 may further associate and store a threshold value of the image feature amount. Here, an image feature amount refers to a feature amount of a tumor which is obtained without using a machine learning model and which is expressed as a numerical value mechanically calculatable from a medical image. Hundreds of image feature amounts are known as such a type of image feature amount, and these feature amounts may include, for example, a tumor area/volume ratio, a tumor heterogeneity (distribution of pixel values), and a tumor contrast variation. Image feature amounts of this type are also known to correlate with gene mutations. For example, a tumor area/volume ratio is known to correlate with a mutation in the gene KRAS, which is a gene related to cell proliferation. Also for example, a tumor heterogeneity is known to correlate with a mutation in the gene ALK, which is a gene related to cell division. For example, a contrast variation is known to correlate with a mutation in the gene RET.

In addition to the configuration described above, the processing circuitry 21 has a collation function 215. The collation function 215 calculates a first image feature amount from the first medical image and a second image feature amount from the second medical image. The collation function 215 specifies a gene corresponding to the first image feature amount from the correlation database 30, and specifies a gene corresponding to the second image feature amount from the correlation database 30. The collation function 215 also calculates a consistency score by collating a third gene mutation classification result corresponding to the first image feature amount of the first medical image with the first gene mutation classification result and by collating a fourth gene mutation classification result corresponding to the second image feature amount of the second medical image with the second gene mutation classification result. Here, the collation function 215 calculates the consistency score with a given fixed value added if both the collation results indicate a match. If at least one of the collation results indicates a mismatch, the collation function 215 calculates the consistency score with the fixed value subtracted. Such a fixed value is a positive value. The fixed value may be called a "reward value". Note that the collation function 215 calculating the consistency score may be understood as correction to the consistency score Gcs_t,n calculated in step ST33. The collation function 215 may be implemented as a part of the judgment function 213 described above. The collation function 215 is an example of a collation unit.

Other configurations are the same as those of any of the first to fourth embodiments.

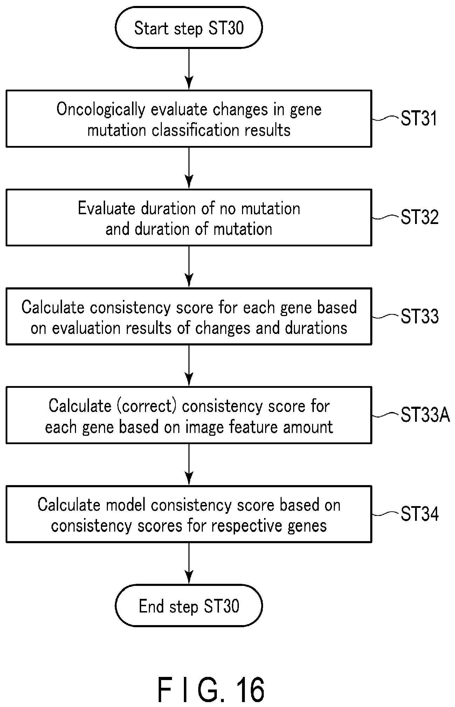

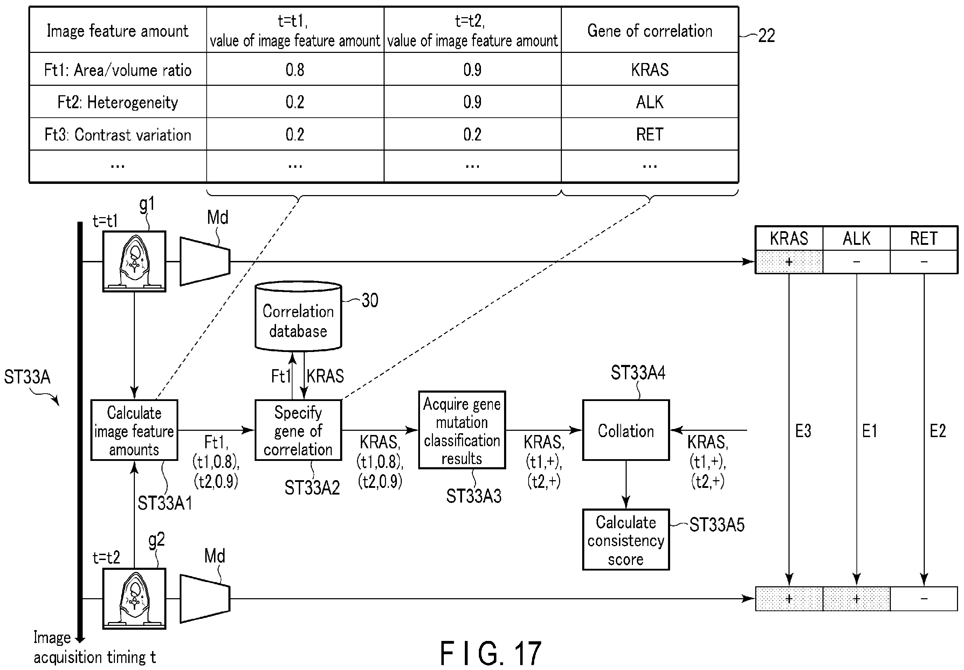

According to the above configuration, after the consistency score Gcs_t,n is calculated for each gene by executing steps ST10 to ST33 as described above, step ST33A as shown in FIGS. 16 and 17 is started. In step ST33A, the processing circuitry 21 calculates (corrects) the consistency score Gcs_t,n for each gene based on image feature amounts of the medical images. For example, the processing circuitry 21 calculates the consistency score by collating the above-discussed change with a change between the third gene mutation classification result corresponding to the image feature amount of the radiation image g1 of time phase t1 and the fourth gene mutation classification result corresponding to the image feature amount of the radiation image g2 of time phase t2. Such a step ST33A includes steps ST33A1 to ST33A5.

In step ST33A1, the processing circuitry 21 calculates the first image feature amount from the radiation image g1 of the first time phase t1 and the second image feature amount from the radiation image g2 of the second time phase t2. For example, the processing circuitry 21 calculates, as the image feature amount, a tumor area/volume ratio Ft1, a tumor heterogeneity Ft2, a contrast variation Ft3, etc., in the manner as shown in the following equations (2) to (4). Note, however, that the image feature amounts are not limited to these, and any feature amounts which can be calculated from medical images may be discretionarily employed. Such discretionary feature amounts are not limited to feature amounts differing in type from these Ft1 to Ft3, but may also be feature amounts of the same type calculated from calculation formulae other than these Ft1 to Ft3.

$$Ft1 = \frac{A}{V}$$

$$\cdots (2)$$

Here, A represents a surface area and V represents a volume.

$$Ft2 = \sum_{i=1}^{N_g} \sum_{j=1}^{N_g} \frac{p(i,j)}{1+|i-j|^2}$$

$$\cdots (3)$$

Here, Ng represents a discrete intensity level of the pixel value. For the discrete intensity level, 1, 2, 3, ..., 255 are generally used. p(i,j) represents a pixel value at the x-coordinate i and the y-coordinate j.

$$Ft3 = \sum_{i=1}^{N_g} \sum_{j=1}^{N_g} (i-j)^2 p(i,j)$$

$$\cdots (4)$$

The processing circuitry 21 stores values of the area/volume ratio Ft1, 0.8 and 0.9, calculated from the respective radiation images g1 and g2 of time phases t1 and t2 in the memory 22 in association with the time phases t1 and t2 and the image feature amount (Ft1). Likewise, the processing circuitry 21 stores values of the heterogeneity Ft2, 0.2 and 0.9, calculated from the respective radiation images g1 and g2 of time phases t1 and t2 in the memory 22 in association with time phases t1 and t2 and the image feature amount (Ft2). Further, the processing circuitry 21 likewise stores values of the contrast variation Ft3, 0.2 and 0.2, calculated from the respective radiation images g1 and g2 of time phases t1 and t2 in the memory 22 in association with time phases t1 and t2 and the image feature amount (Ft3).

After step ST33A1, in step ST33A2, the processing circuitry 21 specifies a gene corresponding to the calculated image feature amount from the correlation database 30. For example, the processing circuitry 21 specifies the gene KRAS corresponding to the area/volume ratio Ft1 from the correlation database 30. Likewise, the processing circuitry 21 specifies the gene ALK corresponding to the heterogeneity Ft2 from the correlation database 30. Further, the processing circuitry 21 likewise specifies the gene RET corresponding to the contrast variation Ft3 from the correlation database 30.

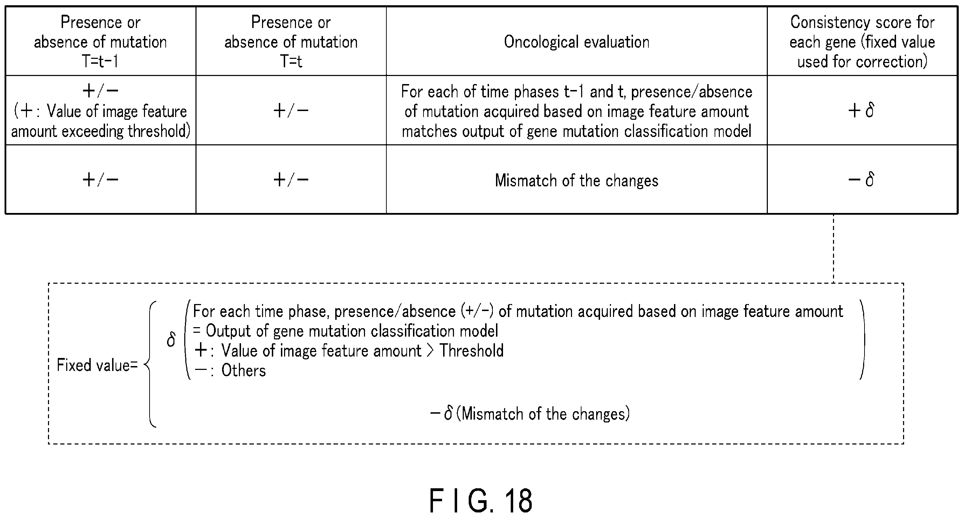

After step ST33A2, in step ST33A3, the processing circuitry 21 generates gene mutation classification results from the image feature amounts for time phases t1 and t2. For example, as shown in FIG. 18, the processing circuitry 21 generates a gene mutation classification result representing the presence of a mutation, "+", if the value of the image feature amount exceeds a threshold value. Also, the processing circuitry 21 generates a gene mutation classification result representing the absence of a mutation, "-", if the value of the image feature amount is less than the threshold value. In the example shown in FIG. 17, the processing circuitry 21 generates a gene mutation classification result representing the presence of a mutation, "+", for each of the values 0.8 and 0.9 of the area/volume ratio Ft1 for time phases t1 and t2 and the value 0.9 of the heterogeneity Ft2 for time phase t2. The processing circuitry 21 also generates a gene mutation classification result representing the absence of a mutation, "-", for each of the value 0.2 of the heterogeneity Ft2 for time phase t1 and the values 0.2 and 0.2 of the contrast variation Ft3 for time phases t1 and t2.

After step ST33A3, in step ST33A4, the processing circuitry 21 collates, for example, the gene mutation classification result "+" generated from the image feature amount for time phase t1 with the gene mutation classification result "+" generated by the gene mutation classification model Md for time phase t1, for the gene KRAS. Also for example, the processing circuitry 21 collates the gene mutation classification result "+" generated from the image feature amount for time phase t2 with the gene mutation classification result "+" generated by the gene mutation classification model Md for time phase t2, for the gene KRAS. Such collation is also performed for the genes ALK and RET in a similar manner.

In step ST33A5, the processing circuitry 21 calculates, for each gene, the consistency score $Gcs\_t,n$ by adding a fixed value $\delta$ to the consistency score $Gcs\_t,n$ acquired in step ST33 as shown in FIG. 18, if both the collation results indicate a match. If at least one of the collation results indicates a mismatch, the processing circuitry 21 calculates the consistency score $Gcs\_t,n$ by subtracting the fixed value $\delta$ from the consistency score $Gcs\_t,n$ acquired in step ST33, for each gene. Note that no limitation is intended by these, and the fixed value $\delta$ to be added and the fixed value $\delta$ to be subtracted may be different from each other. Step ST33A which includes steps ST33A1 to ST33A5 is thus complete.

After completion of step ST33A, the processing circuitry 21 performs step ST34 and the subsequent step in the same manner as described above.

According to the fifth embodiment as described above, the processing circuitry 21 calculates the consistency score by collating the third gene mutation classification result corresponding to the first image feature amount of the first medical image with the first gene mutation classification result and by collating the fourth gene mutation classification result corresponding to the second image feature amount of the second medical image with the second gene mutation classification result. Therefore, in addition to the aforementioned effects, the consistency score for each gene can be more accurately calculated by using a configuration in which the image feature amount having a correlation with a specific gene mutation is reflected on the consistency score calculation. In addition, the judgment of the consistency of a gene mutation classification model can provide a judgment result that also takes into account the image feature amounts.

Also according to the fifth embodiment, the processing circuitry calculates the consistency score with a given fixed value added if both the collation results indicate a match. Therefore, in addition to the aforementioned effects, the calculation of the consistency score for each gene can provide a calculation result that is also supported by image feature amounts. In addition, the judgment of the consistency of a gene mutation classification model can provide a judgment result that is also supported by image feature amounts.

According to the fifth embodiment, the processing circuitry calculates the consistency score with a given fixed value subtracted if at least one of the collation results indicates a mismatch. Therefore, in addition to the aforementioned effects, the calculation of the consistency score for each gene can provide a calculation result that takes into account the deduction due to a mismatch with an image feature amount. In addition, the judgment of the consistency of a gene mutation classification model can provide a judgment result that includes the deduction due to a mismatch with an image feature amount.

(Modifications)

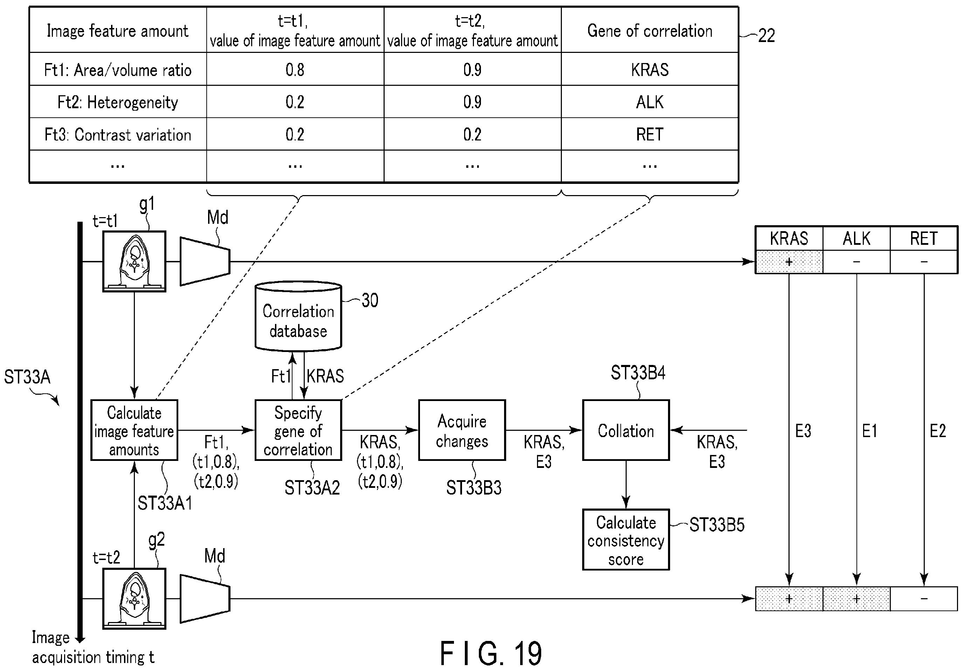

The fifth embodiment has assumed collating, for each time phase, a gene mutation classification result generated by the gene mutation classification model Md with a gene mutation classification result obtained based on an image feature amount, but the embodiment is not limited to this. For example, the collation function 215 of the processing circuitry 21 may calculate the consistency score by collating the above-discussed change with a change between the third gene mutation classification result corresponding to the first image feature amount of the first medical image and the fourth gene mutation classification result corresponding to the second image feature amount of the second medical image. The above-discussed change refers to the change between the first gene mutation classification result and the second gene mutation classification result generated by the generation function 212. In this modification, steps ST33B3 to ST33B5 as shown in FIG. 19 are performed instead of steps ST33A3 to ST33A5.

More specifically, after step ST33A2, in step ST33B3, the processing circuitry 21 generates gene mutation classification results from the image feature amounts for time phases t1 and t2, as described above. That is, the processing circuitry 21 generates a gene mutation classification result representing the presence of a mutation, "+", if the value of the image feature amount exceeds a threshold value. If the value of the image feature amount is less than the threshold value, the processing circuitry 21 generates a gene mutation classification result representing the absence of a mutation, "-". Then, the processing circuitry 21 acquires a change in the gene mutation classification results between two time phases t1 and t2. The change in the gene mutation classification results between two time phases is one of E1: acquisition of a mutation, E2: continuation of no mutation, E3: continuation of a mutation, and E4: extinction of a mutation. That is, for the gene KRAS corresponding to the area/volume ratio Ft1, the processing circuitry 21 acquires E3 as the change in the gene mutation classification results between two time phases t1 and t2. Such acquisition of a change is performed in the same way for the genes ALK and RET corresponding to other image feature amounts.

After step ST33B3, in step ST33B4, the processing circuitry 21 collates the change in the gene mutation classification results obtained from the image feature amounts in step ST33B3 with the change in the gene mutation classification results oncologically evaluated in step ST31. For example, for the gene KRAS, the processing circuitry 21 collates the change E3 obtained in step ST33A3 with the change E3 evaluated in step ST31 for the gene mutation classification results of time phases t1 and t2. Such collation is performed in the same way for the genes ALK and RET.

In step ST33B5, if the result of the collation shows that the changes match each other, the processing circuitry 21 calculates the consistency score Gcs_t,n by adding a fixed value $\delta$ to the consistency score Gcs_t,n acquired in step ST33. If the result of the collation shows that the changes do not match each other, the processing circuitry 21 calculates the consistency score Gcs_t,n by subtracting the fixed value $\delta$ from the consistency score Gcs_t,n acquired in step ST33. Note that no limitation is intended by these, and the fixed value $\delta$ to be added and the fixed value $\delta$ to be subtracted may be different from each other. Step ST33A which includes steps ST33A1 to ST33B5 is thus complete.

After completion of step ST33A, the processing circuitry 21 performs step ST34 and the subsequent step in the same manner as described above. According to such a modification, as in the fifth embodiment, the consistency score for each gene can be more accurately calculated by using a configuration in which a change in the image feature amount having a correlation with a specific gene mutation is reflected on the consistency score calculation. In addition, the judgment of the consistency of a gene mutation classification model can provide a judgment result that also takes into account a change in the image feature amount.

According to at least one embodiment described above, the consistency of a gene mutation classification model can be evaluated.

The term "processor" used in the above description means, for example, a central processing unit (CPU), a graphics processing unit (GPU), or a circuit such as an application specific integrated circuit (ASIC), a programmable logic device, etc. Examples of a programmable logic device include a simple programmable logic device (SPLD), a complex programmable logic devices (CPLD), a field programmable gate array (FPGA), etc. The processor reads and executes a program or programs stored in the memory to realize intended functions. If, for example, the processor is a CPU, the processor reads and executes the program or programs stored in storage circuitry to realize the functions. If, for example, the processor is an ASIC, the functions are directly incorporated into circuitry of the processor in the form of a logic circuit, instead of corresponding programs being stored in storage circuitry. Each processor in the embodiments, etc. is not limited to a single circuit-type processor, and multiple independent circuits may be combined as one processor to realize the intended functions. Furthermore, multiple components or features as given in FIGS. 1 and 10 may be integrated into one processor to realize their functions.

While certain embodiments have been described, these embodiments have been presented by way of example only, and are not intended to limit the scope of the inventions. Indeed, the novel embodiments described herein may be embodied in a variety of other forms; furthermore, various omissions, substitutions, and changes in the form of the embodiments described herein may be made without departing from the spirit of the inventions. The accompanying claims and their equivalents are intended to cover such forms or modifications as would fall within the scope and spirit of the inventions.

**Claims**

1. A medical information processing apparatus comprising processing circuitry configured to:

   acquire a first medical image and a second medical image acquired in at least a first time phase and a second time phase;
   input the first medical image and the second medical image into a gene mutation classification model to generate a first gene mutation classification result and a second genetic mutation classification result; and
   judge consistency of the gene mutation classification model based on the first gene mutation classification result and the second gene mutation classification result.

2. The medical information processing apparatus according to claim 1, wherein the processing circuitry is configured to judge the consistency of the gene mutation classification model based further on medical validity of a gene mutation.

3. The medical information processing apparatus according to claim 2, wherein the processing circuitry is configured to judge the consistency by evaluating a change between the first gene mutation classification result and the second gene mutation classification result based on the validity.

4. The medical information processing apparatus according to claim 3, wherein

   the validity includes that: it is medically valid that a tumor acquires a new gene mutation while continuing an existing mutation; it is medically valid that a gene mutation of a tumor continues with increasing complexity; and extinction of a gene mutation in a tumor is rare and not medically valid, and
   the change represents, for each gene, acquisition of a mutation, continuation of no mutation, continuation of a mutation, and extinction of a mutation.

5. The medical information processing apparatus according to claim 4, wherein the processing circuitry is configured to judge the consistency by evaluating a degree of conformance of the change to the validity and calculating a consistency score for each gene.

6. The medical information processing apparatus according to claim 5, wherein the processing circuitry is configured to, if the change is continuation of no mutation or continuation of a mutation, calculate the consistency score to have a positive value corresponding to a duration for which no mutation or the mutation has continued.

7. The medical information processing apparatus according to claim 5, wherein the processing circuitry is configured to, if the change is extinction of a mutation, calculate the consistency score to have a negative value corresponding to a duration for which the mutation has continued.

8. The medical information processing apparatus according to claim 5, wherein the processing circuit is configured to, if the change is acquisition of a mutation, calculate the consistency score based on a predetermined positive value.

9. The medical information processing apparatus according to any one of claims 5 to 8, wherein the processing circuitry is configured to calculate a model consistency score for the gene mutation classification model by summing up the consistency scores for respective genes, and to judge the consistency based on the model consistency score and a threshold value.

10. The medical information processing apparatus according to any one of claims 1 to 8, wherein the processing circuitry is configured to withhold judgment of the consistency if gene mutation treatment is performed between the first time phase and the second time phase.

11. The medical information processing apparatus according to any one of claims 1 to 8, wherein the processing circuitry is configured to

    generate the first gene mutation classification result and the second gene mutation classification result for each of a plurality of gene mutation classification models,
    judge the consistency for each of the plurality of gene mutation classification models, and
    select a gene mutation classification model having said consistency from among the plurality of gene mutation classification models based on a result of judgment of the consistency.

12. The medical information processing apparatus according to any one of claims 1 to 8, wherein the processing circuitry is configured to generate a medical image by inputting, into an image generation model, a medical image acquired in a time phase corresponding to that of a missing one of the first medical image and the second medical image, and acquire the generated medical image as the missing one of the first medical image and the second medical image.

13. The medical information processing apparatus according to any one of claims 1 to 8, wherein the processing circuitry is configured to calculate the consistency score by collating a third gene mutation classification result corresponding to a first image feature amount of the first medical image with the first gene mutation classification result and collating a fourth gene mutation classification result corresponding to a second image feature amount of the second medical image with the second gene mutation classification result.

14. A medical information processing method comprising:

acquiring a first medical image and a second medical image acquired in at least a first time phase and a second time phase;
inputting the first medical image and the second medical image into a gene mutation classification model to generate a first gene mutation classification result and a second gene mutation classification result; and
judging consistency of the gene mutation classification model based on the first gene mutation classification result and the second gene mutation classification result.

15. A non-transitory computer-readable storage medium storing computer-executable instructions, wherein the instructions, if executed by a processor, cause the processor to perform a method comprising:

acquiring a first medical image and a second medical image acquired in at least a first time phase and a second time phase;
inputting the first medical image and the second medical image into a gene mutation classification model to generate a first gene mutation classification result and a second gene mutation classification result; and
judging consistency of the gene mutation classification model based on the first gene mutation classification result and the second gene mutation classification result.

Medical information processing apparatus — 20

Memory — 22

Input interface — 23

Display — 24

Communication interface — 25

Processing circuitry — 21

Acquisition function — 211

Generation function — 212

Judgment function — 213

Image database — 10

F I G. 1

Start

Acquire radiation images of multiple time phases — ST10

Execute gene mutation classification model for each radiation image of respective time phases — ST20

Calculate model consistency score based on gene mutation classification results for respective time phases — ST30

Judge consistency of gene mutation classification model based on model consistency score — ST40

End

F I G. 2

Start step ST30

Oncologically evaluate changes in gene
mutation classification results ⌐ST31

Evaluate duration of no mutation
and duration of mutation ⌐ST32

Calculate consistency score for each gene based
on evaluation results of changes and durations ⌐ST33

Calculate model consistency score based on
consistency scores for respective genes ⌐ST34

End step ST30

# F I G. 3

F I G. 4

EP 4 414 997 A1

|  | KRAS | ALK | RET | ROS1 | TP53 | EGFR | BRAF |
|---|---|---|---|---|---|---|---|
| E1: Acquisition of mutation |  | Once |  | Once |  | Once | Once |
| E2: Continuation of no mutation |  |  | t3−t1 | t2−t1 | t3−t1 |  |  |
| E3: Continuation of mutation | t3−t1 | t3−t2 |  |  |  | t3−t2 |  |
| E4: Extinction of mutation |  |  |  |  |  |  | t3−t2 |

# F I G. 5

| Presence or absence of mutation t=T−1 | Presence or absence of mutation t=T | Oncological evaluation | Consistency score for each gene |
|---|---|---|---|
| − | + | E1: Acquisition of mutation | +0.1 : Acquisition of mutation and increase in complexity are oncologically valid |
| − | − | E2: Continuation of no mutation | +0.2×(weight according to duration of −): Gene that is not easily mutated is weighted |
| + | + | E3: Continuation of mutation | +0.2×(weight according to duration of +): Gene that has long survival period contributes to tumor survival and is weighted according to period |
| + | − | E4: Extinction of mutation | −0.5×(weight according to duration of +) : Extinction of gene that has long survival period is oncologically rare, and a larger penalty according to period is given |

F I G. 6

EP 4 414 997 A1

| Presence or absence of mutation t=T-1 | Presence or absence of mutation t=T | Oncological evaluation | Consistency score for each gene |
|---|---|---|---|
| − | + | E1: Acquisition of mutation | +0.1 : Acquisition of mutation and increase in complexity are oncologically valid |
| − | − | E2: Continuation of no mutation | +0.2×(weight according to duration of −): Gene that is not easily mutated is weighted |
| + | + | E3: Continuation of mutation | +0.2×(weight according to duration of +): Gene that has long survival period contributes to tumor survival and is weighted according to period |
| + | − | E4: Extinction of mutation | −0.5×(weight according to duration of +) : Extinction of gene that has long survival period is oncologically rare, and a larger penalty according to period is given |
| + (Gene determined to involve mutation by gene test) | + | Output coinciding with definitive diagnosis | +0.2 × (larger weight according to duration of +): Gene that has long survival period contributes to tumor survival and is more largely weighted according to period |
| −/+ (Gene determined to involve mutation by gene test) | − | Contradiction to definitive diagnosis | Negative infinity |
| − (Gene confirmed to have co-occurrence characteristic with already mutated gene) | + | Match with known co-occurrence relationship | $+\beta$ |
| − (Gene confirmed to have exclusivity relationship to already mutated gene) | + | Mismatch with known exclusivity relationship | $-\beta$ |

F I G. 7

EP 4 414 997 A1

Start step ST40

ST41

Does model
consistency score exceed
threshold? — No

Yes

ST42

Does model
consistency score increase from previous
time phase? — No

Yes

ST44

Does time phase
of model consistency score
follow treatment-involved
time phase? — No

Yes

ST43

Determine consistency

ST45

Withhold consistency judgment

ST46

Determine lack of
consistency

End step ST40

F I G. 8

FIG. 9

t=t11 g11

t=t12 g12

t=t13

t=t14 g14

t=t15 g15

Molecular target drug administration

Md

KRAS
ALK
RET
ROS1
TP53
EGFR
BRAF

E4

E4

Mcs(t11)=0.70

Mcs(t12)=0.71

Mcs(t14)=0.39

Mcs(t15)=0.77

ST43 Determine consistency

ST43 Determine consistency

ST45 Withhold consistency judgment

ST43 Determine consistency

F I G. 10

EP 4 414 997 A1

```
                    ┌──────────┐
                    │  Start   │
                    └────┬─────┘
                         ▼
┌ ─ ─ ─ ─ ─ ─ ─ ─ ─ ─ ─ ─ ─ ─ ─ ─ ─ ─ ─ ─ ┐
   ┌──────────────────────────────┐
│  │   Prepare multiple gene       │ ─── ST2  │
   │   mutation                    │
│  │   classification models       │         │
   └───────────────┬──────────────┘
└ ─ ─ ─ ─ ─ ─ ─ ─ ─│─ ─ ─ ─ ─ ─ ─ ─ ─ ─ ─ ─ ┘
                   ▼
   ┌──────────────────────────────────┐
   │ Acquire radiation images of       │ ─── ST10
   │ multiple time phases              │
   └───────────────┬──────────────────┘
                   ▼
   ┌──────────────────────────────────┐
   │ Execute gene mutation classification │ ─── ST20
   │ model for each radiation image of    │
   │ respective time phases               │
   └───────────────┬──────────────────┘
                   ▼
   ┌──────────────────────────────────┐
   │ Calculate model consistency score │ ─── ST30
   │ based on gene mutation            │
   │ classification results for        │
   │ respective time phases            │
   └───────────────┬──────────────────┘
                   ▼
   ┌──────────────────────────────────┐
   │ Judge consistency of gene mutation │ ─── ST40
   │ classification model based on      │
   │ model consistency score            │
   └───────────────┬──────────────────┘
                   ▼
```

ST51

Have all gene mutation classification models been judged?

No — Yes

Select gene mutation classification model based on judgment results — ST52

End

F I G. 11

α=0.6

t=t1,···,tm

ST52

Md1

Gene mutation classification model trained with CT images of Asian subjects

Mcs(tm)=0.4

ST46

Determine lack of consistency

×

CT images of African subject

○

Md2

Gene mutation classification model trained with CT images of European subjects

Mcs(tm)=0.7>α

ST43

Determine consistency

Radiation images of multiple time phases

gt

×

Not using gene mutation classification models

F I G. 12

```
        ┌─────────────────────┐
        │   Start step ST10   │
        └─────────────────────┘
                   │
                   ▼
   ┌──────────────────────────────────┐
   │  Acquire radiation images of time │──── ST11
   │   phases available for acquisition│
   └──────────────────────────────────┘
                   │
                   ▼
              ╱─────────╲                    ST12
            ╱             ╲                No
   ╱─────────────────────────────────╲────────────┐
   ╲   Is there missing radiation image?╱          │
            ╲             ╱                         │
              ╲─────────╱                           │
                   │ Yes                            │
                   ▼                                │
   ┌──────────────────────────────────┐            │
   │   Retrieve noise information, patient│──── ST13 │
   │  information, and ultrasound image │            │
   └──────────────────────────────────┘            │
                   │                                │
                   ▼                                │
   ┌──────────────────────────────────┐            │
   │ Execute image generation model based on noise│── ST14 │
   │information, patient information, and ultrasound image│ │
   └──────────────────────────────────┘            │
                   │                                │
                   ▼                                │
   ┌──────────────────────────────────┐            │
   │   Acquire generated radiation image│──── ST15   │
   │      as missing radiation image   │            │
   └──────────────────────────────────┘            │
                   │                                │
                   ▼◄───────────────────────────────┘
        ┌─────────────────────┐
        │    End step ST10    │
        └─────────────────────┘
```

F I G. 13

ST14,ST15

Md_G

Noise information → Image generation model

Patient information →

Ultrasound image →

gG

→ Radiation image

# F I G. 14

F I G. 15

Start step ST30

Oncologically evaluate changes in gene
mutation classification results —— ST31

Evaluate duration of no mutation
and duration of mutation —— ST32

Calculate consistency score for each gene based
on evaluation results of changes and durations —— ST33

Calculate (correct) consistency score for
each gene based on image feature amount —— ST33A

Calculate model consistency score based on
consistency scores for respective genes —— ST34

End step ST30

F I G. 16

EP 4 414 997 A1

| Image feature amount | t=t1, value of image feature amount | t=t2, value of image feature amount | Gene of correlation | |
|---|---|---|---|---|
| Ft1: Area/volume ratio | 0.8 | 0.9 | KRAS | ~22 |
| Ft2: Heterogeneity | 0.2 | 0.9 | ALK | |
| Ft3: Contrast variation | 0.2 | 0.2 | RET | |
| ... | ... | ... | ... | |

ST33A

t=t1  g1  Md

| KRAS | ALK | RET |
|---|---|---|
| + | − | − |

Correlation database ~30

Ft1  KRAS

Calculate image feature amounts
ST33A1

Ft1, (t1,0.8), (t2,0.9)

Specify gene of correlation
ST33A2

KRAS, (t1,0.8), (t2,0.9)

Acquire gene mutation classification results
ST33A3

KRAS, (t1,+), (t2,+)

Collation
ST33A4

KRAS, (t1,+), (t2,+)

Calculate consistency score —ST33A5

E3  E1  E2

t=t2  g2  Md

| + | + | − |
|---|---|---|

Image acquisition timing t

F I G. 17

| Presence or absence of mutation T=t−1 | Presence or absence of mutation T=t | Oncological evaluation | Consistency score for each gene (fixed value used for correction) |
|---|---|---|---|
| +/− (+: Value of image feature amount exceeding threshold) | +/− | For each of time phases t−1 and t, presence/absence of mutation acquired based on image feature amount matches output of gene mutation classification model | +δ |
| +/− | +/− | Mismatch of the changes | −δ |

Fixed value= $\begin{cases} \delta \begin{cases} \text{For each time phase, presence/absence (+/−) of mutation acquired based on image feature amount} \\ \text{= Output of gene mutation classification model} \\ \text{+: Value of image feature amount > Threshold} \\ \text{−: Others} \end{cases} \\ \\ -\delta \text{ (Mismatch of the changes)} \end{cases}$

F I G. 18

| Image feature amount | t=t1, value of image feature amount | t=t2, value of image feature amount | Gene of correlation |
|---|---|---|---|
| Ft1: Area/volume ratio | 0.8 | 0.9 | KRAS |
| Ft2: Heterogeneity | 0.2 | 0.9 | ALK |
| Ft3: Contrast variation | 0.2 | 0.2 | RET |
| ... | ... | ... | ... |

~22

| KRAS | ALK | RET |
|---|---|---|
| + | − | − |

| KRAS | ALK | RET |
|---|---|---|
| + | + | − |

t=t1  g1  Md

Correlation database ~30

Ft1 | KRAS

ST33A

Calculate image feature amounts

Ft1, (t1,0.8), (t2,0.9)

ST33A1

Specify gene of correlation

ST33A2

KRAS, (t1,0.8), (t2,0.9)

Acquire changes

ST33B3

KRAS, E3

ST33B4

Collation

KRAS, E3

Calculate consistency score — ST33B5

E3  E1  E2

t=t2  g2  Md

Image acquisition timing t

# F I G. 19

Europäisches
Patentamt

European
Patent Office

Office européen
des brevets

# EUROPEAN SEARCH REPORT

Application Number

EP 23 20 8093

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MOON WOO KYUNG ET AL: "Evaluation of TP53/PIK3CA mutations using texture and morphology analysis on breast MRI", MAGNETIC RESONANCE IMAGING, ELSEVIER SCIENCE, TARRYTOWN, NY, US, vol. 63, 16 August 2019 (2019-08-16), pages 60-69, XP085909730, ISSN: 0730-725X, DOI: 10.1016/J.MRI.2019.08.026 [retrieved on 2019-08-16] * whole document, in particular abstract; Materials; Tables and Figures * ----- | 1-15 | INV. G16H30/40 G16H50/20 |
| A | QI YANA ET AL: "The application of radiomics in predicting gene mutations in cancer", EUROPEAN RADIOLOGY, SPRINGER BERLIN HEIDELBERG, BERLIN/HEIDELBERG, vol. 32, no. 6, 20 January 2022 (2022-01-20), pages 4014-4024, XP037839390, DOI: 10.1007/S00330-021-08520-6 [retrieved on 2022-01-20] * whole document, in particular abstract; introduction; Tables; Limitations and Conclusion * ----- | 1-15 | |
| A | WO 2022/241300 A1 (GENENTECH INC [US]; HOFFMANN LA ROCHE [CH]; HOFFMANN LA ROCHE [US]) 17 November 2022 (2022-11-17) * whole document, in particular abstract and claims * ----- | 1-15 | |

**TECHNICAL FIELDS SEARCHED (IPC)**

G16H

The present search report has been drawn up for all claims

| Place of search | Date of completion of the search | Examiner |
|---|---|---|
| Munich | 8 March 2024 | Vanmontfort, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

......................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 23 20 8093

This annex lists the patent family members relating to the patent documents cited in the above-mentioned European search report.
The members are as contained in the European Patent Office EDP file on
The European Patent Office is in no way liable for these particulars which are merely given for the purpose of information.

08-03-2024

| Patent document cited in search report | Publication date | Patent family member(s) | Publication date |
|---|---|---|---|
| WO 2022241300 A1 | 17-11-2022 | EP 4338172 A1 | 20-03-2024 |
| | | WO 2022241300 A1 | 17-11-2022 |

EPO FORM P0459

For more details about this annex : see Official Journal of the European Patent Office, No. 12/82